# EUROPÄISCHE PATENTSCHRIFT

(11) **EP 1 762 233 B1**
(45) Veröffentlichungstag und Bekanntmachung des Hinweises auf die Patenterteilung: **26.03.2008**
(21) Anmeldenummer: 06090157.6
(22) Anmeldetag: 08.09.2006
(51) Int. Cl.: A61K 31/00, A61P 39/06, A61K 31/355, A61K 33/04, A61K 31/375, A61K 33/06, A61K 33/30, A61K 31/455, A61K 31/51, A61K 31/4415, A61K 31/525, A61K 31/122, A61K 31/195

(54) **Mittel zur Prävention von Sauerstoff-induzierten Schäden beim Tauchen**
Preventive agent for oxygen-induced damages from diving
Agent préventif des lésions induites par l'oxygène pendant la plongée sous-marine

(30) Priorität: 08.09.2005 DE 102005043403
(43) Veröffentlichungstag der Anmeldung: 14.03.2007
(73) Patentinhaber: Schielein, Felix, 90439 Nürnberg (DE)
(72) Erfinder: Schielein, Felix, 90439 Nürnberg (DE)
(74) Vertreter: Maikowski, Michael

(56) Entgegenhaltungen:
- DE-U1- 20 116 346
- IKEDA MAKOTO ET AL: "Supplementation of antioxidants prevents oxidative stress during a deep saturation dive" TOHOKU JOURNAL OF EXPERIMENTAL MEDICINE, Bd. 203, Nr. 4, August 2004 (2004-08), Seiten 353-357, XP002415412 ISSN: 0040-8727
- SUREDA A ET AL: "Hypoxia/reoxygenation and vitamin c intake influence no synthesis and antioxidant defenses of neutrophils" FREE RADICAL BIOLOGY AND MEDICINE, ELSEVIER SCIENCE, XX, Bd. 37, Nr. 11, 1. Dezember 2004 (2004-12-01), Seiten 1744-1755, XP004622792 ISSN: 0891-5849
- MONEY D F ET AL: "Under-water diving, oxygen poisoning and vitamin E." THE NEW ZEALAND MEDICAL JOURNAL. JAN 1972, Bd. 75, Nr. 476, Januar 1972 (1972-01), Seiten 34-35, XP009077358 ISSN: 0028-8446
- DATABASE BIOSIS [Online] BIOSCIENCES INFORMATION SERVICE, PHILADELPHIA, PA, US; 1991, DENG X: "STUDY OF NUTRITION IN DIVERS DURING HELIUM-NITROGEN-OXYGEN SIMULATED SATURATION DIVING AT THE DEPTH OF 300M" XP002415427 Database accession no. PREV199293118502 & ACTA NUTRIMENTA SINICA, Bd. 13, Nr. 3, 1991, Seiten 202-207, ISSN: 0512-7955
- HINTON P S ET AL: "Nutrient intakes and dietary behaviors of male and female collegiate athletes" INTERNATIONAL JOURNAL OF SPORT NUTRITION AND EXERCISE METABOLISM 2004 UNITED STATES, Bd. 14, Nr. 4, 2004, Seiten 389-405, XP009077227 ISSN: 1526-484X
- PETERSEN H L ET AL: "Body composition, dietary intake, and iron status of female collegiate swimmers and divers." INTERNATIONAL JOURNAL OF SPORT NUTRITION AND EXERCISE METABOLISM 16 (3) 2006, 2006, XP009077293

## Beschreibung

Die Erfindung betrifft Arzneimittel, durch welche bei Tauchern chronische Schäden durch Exposition gegenüber erhöhten Sauerstoff-Partialdrücken bei Tauchgängen vermieden werden.

Allgemein hat jedes auf oxidativem Metabolismus aufbauende Lebewesen biochemisch eine Balance zwischen der für die Aufrechterhaltung der Lebensvorgänge notwendigen Zufuhr von Sauerstoff in alle Gewebe einerseits, und dem Schutz eben dieser Gewebe vor unkontrollierter Oxidation von Gewebekomponenten andererseits zu wahren. Eine Reihe von Mechanismen gewährleistet diesen Schutz, z.B. Enzyme wie Superoxid-Dismutase, oder die Anwesenheit von Antioxidantien und Radikalfängern im Gewebe. Diese Mechanismen sind beim Menschen evolutionär auf eine Sauerstoffkonzentration optimiert, wie sie bei normalem atmosphärischen Druck in Atemluft vorliegt (ca. 210 mbar Sauerstoff-Partialdruck).

Taucher sind während ihres Aufenthalts unter Wasser erhöhtem Druck ausgesetzt. Bei Tauchgängen, während derer der Taucher Atemgas erhält, also beim Gerätetauchen, wie es Sporttaucher betreiben, sowie beim Arbeiten in Tauchglocken oder ähnlichem Gerät, wird dem Taucher Sauerstoff in Form von komprimierter Luft oder Sauerstoff-Inertgasgemischen verabreicht.

Bei steigendem Druck durch die über dem Taucher stehende Wassersäule steigt auch der Partialdruck des Sauerstoffs in dessen Körper. Infolgedessen sind die Gewebe des Tauchers einer höheren Sauerstoff-Konzentration ausgesetzt als physiologisch normal ist. Die Folge dessen ist erhöhter oxidativer Stress.

Erhöhter oxidativer Stress schädigt vor allem Grundgewebe, insbesondere werden Proteoglykan-Strukturen angegriffen, die für ein reibungsloses Funktionieren vieler biologischer Vorgänge (Immunsystem, Entgiftung, elektrischer Tonus, Informationstransport, Wasserhaushalt u.v.m.) von zentraler Bedeutung sind. Diese Grundgewebestrukturen gilt es zu schützen, um chronische Effekte eines Sauerstoff-Überangebotes zu vermeiden.

Der Umgebungsdruck nimmt beim Tauchen sehr schnell zu. Der Druck des Atemgases steigt mit zunehmender Wassertiefe rasch an. Je 10 m Tauchtiefe erhöht sich der Druck um 1 bar. Daraus folgt, dass ein Taucher in einer Tiefe von 10 m bereits einem Druck von 2 bar, in einer Tiefe von 20 m einem Druck von 3 bar und in 30 m Tiefe einem Druck von 4 bar ausgesetzt ist. Damit erhöht sich der Sauerstoffpartialdruck in Körpergeweben (Oxydose) beträchtlich, d. h. ein Taucher nimmt unverhältnismäßig viel Sauerstoff unter unphysiologisch hohen Druckverhältnissen zu sich. Die vermehrte Sauerstoffzufuhr hat eine überhöhte Radikalbelastung zur Folge. Die Zellen sind nicht in der Lage, diese erhöhte Radikalbelastung alleine abzufangen. Die dadurch bedingten chronischen Schäden an den Proteoglykan-Strukturen des Interstitiums spürt der Taucher nicht sofort. Offensichtliche Erkrankungen, wie z. B. die Zerstörung der Gelenkknorpelsubstanz, spürt der Taucher oft erst Jahre später.

Bei starker Sonneneinstrahlung, Nikotinkonsum, zu wenig Flüssigkeitszufuhr, Alkoholkonsum, zu wenig Erholungsphasen, einseitige Ernährung und Stress wird eine verstärkte Radikalbelastung beobachtet. Taucher sind häufig zusätzlich einer oder mehreren dieser Belastungen ausgesetzt.

Um dem durch die erhöhte Stickstoffzufuhr befürchteten Tiefenrausch oder der Dekompressionskrankheit entgegen zu wirken, werden in rasch zunehmendem Maße Tauchern Atemgaskombinationen zur Verfügung gestellt, bei denen der Sauerstoffgehalt von 21 % auf 36 bis 40 %, in seltenen Fällen sogar auf 60 % erhöht wird.
Derartig sauerstoffreiche und stickstoffarme Atemgase werden als Nitrox-Gase bezeichnet. Das Wort Nitrox ist aus den englischen Bezeichnungen für Stickstoff (Nitrogen) und Sauerstoff (Oxygen) gebildet worden. Das Wort Nitrox bezeichnet keine Gasmischung mit festen prozentualen Gasanteilen, sondern gibt nur Aufschluss über dessen Hauptbestandteile. In diesem Zusammenhang wird auch von "technischem Tauchen" gesprochen.
Es ist möglich, die Radikalbelastung des Gewebes zu messen. So können freie Radikale unter physiologischen Bedingungen mit dem so genannten FORT-Test nachgewiesen werden. Im FORT-Test werden Hydroperoxide (ROOH) als valider Index für freie Radikale erfasst. Die Messergebnisse reichen von 200 FORT-Einheiten, die als physiologisch normaler Wert gelten, bis über 500 FORT-Einheit.

**Tabelle 1**

| | |
|---|---|
| FORT 200 - 310 | Normale Radikalbelastung |
| FORT 311 - 340 | Leichter oxidativer Stress |
| FORT 341 - 400 | Signifikanter oxidativer Stress |
| FORT 401 - 500 | Hoher oxidativer Stress |
| FORT größer 501 | Sehr hoher oxidativer Stress |

Vorarbeiten hinsichtlich der Prävention von durch Tauchgänge induziertem oxidativen Stress sind kaum bekannt. Ikeda et. al (Tohuko J. Exp. Med. 2004, 203, 353-357) beschreiben eine Untersuchung, in der die Leberfunktion bei extremen Tieftauchgängen von 30 Tagen ununterbrochener Dauer auf 400 m Tiefe anhand von Leberenzym-Messungen verfolgt wird. Die Autoren dieser Veröffentlichung berichten, dass tägliche Verabreichung von 0,6 g Ascorbinsäure, 150 mg Tocopherol sowie 0,6 g Tee-Catechine während der Tauchgänge zu einer signifikanten Reduktion des Anstiegs der Enzymwerte führte. Messungen zur Reduktion von chronische Schäden verursachenden Radikalkonzentrationen im Gewebe wurden nicht durchgeführt.

Vor dem Hintergrund dieses Standes der Technik ist es die Aufgabe der vorliegenden Erfindung, geeignete Mittel zur Verfügung zu stellen, um Taucher vor den schädlichen Auswirkungen des erhöhten Sauerstoff-Partialdruckes bei Tauchgängen nach Möglichkeit zu schützen. Gelöst wird diese Aufgabe durch das im Hauptanspruch beschriebene Arzneimittel.

Im Rahmen einer Testreihe wurden 20 Taucher auf einer sechs-tägigen Tauchsafari mit drei Tauchgängen zwischen 20 und 30 m Tauchtiefe pro Tag mit dem FORT-Test getestet. Zehn Taucher gingen ohne oxidativen Schutz in das Experiment. Zehn Taucher (in der folgenden Tabelle (2) Probanden 11-20) wurden mit einem Arzneimittel der Zusammensetzung wie in Beispiel 1 gegeben, versorgt.

**Tabelle 2**

| **Taucher** | **Name** | **Ausgangswert** | **1. Tag** | **2. Tag** | **3. Tag** | **4. Tag** | **5. Tag** | **6. Tag** |
|---|---|---|---|---|---|---|---|---|
| 1 | Manuel B. | 340 | 381 | 387 | 365 | 389 | 403 | 390 |
| 2 | Erich S. | 295 | 380 | 355 | 385 | 400 | 406 | 385 |
| 3 | Hanna R. | 314 | 366 | 377 | 420 | 410 | 408 | 405 |
| 4 | Helmut R. | 348 | 405 | 409 | 440 | 442 | 418 | 451 |
| 5 | Ernst W. | 385 | 424 | 452 | 462 | 485 | 502 | 506 |
| 6 | Wolf H. | 344 | 368 | 398 | 386 | 405 | 409 | 385 |
| 7 | Regina H. | 302 | 305 | 340 | 345 | 342 | 309 | 320 |
| 8 | Bernd L. | 324 | 385 | 376 | 395 | 399 | 402 | 430 |
| 9 | Bernd S. | 301 | 320 | 350 | 345 | 384 | 394 | 387 |
| 10 | Henry O. | 390 | 440 | 445 | 448 | 468 | 425 | 468 |
| 11 | Bea F. | 295 | 310 | 321 | 312 | 305 | 324 | 320 |
| 12 | Ralf F. | 311 | 320 | 324 | 310 | 315 | 319 | 340 |
| 13 | Kerstin D. | 302 | 305 | 308 | 304 | 299 | 289 | 310 |
| 14 | Bernd D. | 320 | 320 | 325 | 324 | 330 | 318 | 330 |
| 15 | Benno F. | 314 | 320 | 315 | 319 | 342 | 335 | 330 |
| 16 | Charly R. | 291 | 310 | 324 | 315 | 312 | 310 | 314 |
| 17 | Michael S. | 295 | 298 | 310 | 312 | 315 | 304 | 300 |
| 18 | Gerd S. | 240 | 264 | 268 | 265 | 245 | 274 | 280 |
| 19 | Jonas G. | 267 | 285 | 295 | 294 | 301 | 322 | 324 |
| 20 | Karin G. | 302 | 305 | 307 | 304 | 320 | 312 | 314 |

Eine Auswertung der Tabelle zeigt deutlich, dass die mit dem erfindungsgemäßen Arzneimittel zur antioxidativen Therapie versorgten Taucher einen signifikant niedrigeren Anstieg der oxidativen Belastung als die anderen Taucher aufwiesen.

Zahlreiche Versuche mit anderen Zusammensetzungen führten zu dem überraschenden Ergebnis, dass das nachstehend beschriebene Arzneimittel für die Lösung der hier zugrundeliegenden Problematik der Prävention von erhöhten Radikalkonzentrationen im Gewebe nach Tauchgängen die vorteilhafteste Zusammensetzung darstellt.
Erfindungsgemäß wird Tauchern vor dem Tauchgang ein Arzneimittel der folgenden Zusammensetzung zur Verfügung gestellt :

| | |
|---|---|
| Vitamin B1 | 1 bis 10 mg, |
| Vitamin B2 | 1 bis 10 mg, |
| Nikotinamid | 10 bis 100 mg, |
| Vitamin B6 | 1 bis 10 mg, |
| Vitamin C | 0,5 bis 5 g, |
| Vitamin E | 100 bis 1000 i.E., |
| Zink | 50 bis 200 mg, |
| Selen | 50 bis 200 µg, |
| Magnesium | 100 bis 500 mg, |
| L-Cystin | 50 bis 200 mg, |
| Coenzym Q10 | 5 bis 40 mg |

Insbesondere erfindungsgemäß ist ein Arzneimittel der folgenden Zusammensetzung:

| | |
|---|---|
| Vitamin B1 | 3 bis 7 mg, |
| Vitamin B2 | 3 bis 7 mg, |
| Nikotinamid | 30 bis 70 mg, |
| Vitamin B6 | 3 bis 7 mg, |
| Vitamin C | 1,5 bis 3 g, |
| Vitamin E | 400 bis 600 i.E., |
| Zink | 50 bis 200 mg, |
| Selen | 80 bis 150 µg, |
| Magnesium | 100 bis 500 mg, |
| L-Cystin | 80 bis 150 mg, |
| Coenzym Q10 | 10 bis 25 mg |

Zur Erprobung wurden die in den folgenden Beispielen genannten Arzneimittel hergestellt und jeweils an Probanden zur oralen Einnahme gegeben. Die Radikalkonzentration im Gewebe wurde nach mehreren Tieftauchgängen durch FORT-Test ermittelt und mit den entsprechenden Werten von nicht-behandelten Probanden, die ebenfalls das gleiche Druckregime erfahren hatten, verglichen.

### Beispiel 1: Arzneimittel zur Prävention von Schäden durch oxidativem Stress beim Tauchen

| | |
|---|---|
| Vitamin B1 | 5 mg, |
| Vitamin B2 | 5 mg, |
| Nikotinamid | 50 mg, |
| Vitamin B6 | 5 mg, |
| Vitamin C | 3 g, |
| Vitamin E | 500 i.E., |
| Zink | 100 mg, |
| Selen | 100 µg, |
| Magnesium | 300 mg, |
| L-Cystin | 100 mg und |
| Coenzym Q10 | 20 mg |

### Beispiel 2: Arzneimittel zur Prävention von Schäden durch oxidativem Stress beim Tauchen

| | |
|---|---|
| Vitamin B1 | 5 mg, |
| Vitamin B2 | 5 mg, |
| Nikotinamid | 50 mg, |
| Vitamin B6 | 5 mg, |
| Vitamin C | 1,5 g, |
| Vitamin E | 400 i.E., |
| Zink | 100 mg, |
| Selen | 100 µg, |
| Magnesium | 300 mg, |
| L-Cystin | 100 mg und |
| Coenzym Q10 | 20 mg |

## Patentansprüche

1. Arzneimittel zur Prävention von Schäden durch oxidativen Stress beim Tauchen,
enthaltend
pro Verabreichungseinheit
| | |
|---|---|
| Vitamin B1 | 1 bis 10 mg, |
| Vitamin B2 | 1 bis 10 mg, |
| Nikotinamid | 10 bis 100 mg, |
| Vitamin B6 | 1 bis 10 mg, |
| Vitamin C | 0,5 bis 5 g, |
| Vitamin E | 100 bis 1000 i.E., |
| Zink | 50 bis 200 mg, |
| Selen | 50 bis 200 µg, |
| Magnesium | 100 bis 500 mg, |
| L-Cystin | 50 bis 200 mg und |
| Coenzym Q10 | 5 bis 40 mg |

2. Arzneimittel gemäß Anspruch 1, wobei die Mengen pro Verabreichungseinheit sind:
| | |
|---|---|
| Vitamin B1 | 3 bis 7 mg, |
| Vitamin B2 | 3 bis 7 mg, |
| Nikotinamid | 30 bis 70 mg, |
| Vitamin B6 | 3 bis 7 mg, |
| Vitamin C | 1,5 bis 3 g, |
| Vitamin E | 400 bis 600 i.E., |
| Zink | 50 bis 200 mg, |
| Selen | 80 bis 150 µg, |
| Magnesium | 100 bis 500 mg, |
| L-Cystin | 80 bis 150 mg und |
| Coenzym Q10 | 10 bis 25 mg |

## Claims

1. Medicament for preventing damage through oxidative stress during diving
comprising
per administration unit
| | |
|---|---|
| Vitamin B1 | 1 to 10 mg, |
| Vitamin B2 | 1 to 10 mg, |
| Nicotinamide | 10 to 100 mg, |
| Vitamin B6 | 1 to 10 mg, |
| Vitamin C | 0.5 to 5 g, |
| Vitamin E | 100 to 1000 I.U., |
| Zinc | 50 to 200 mg, |
| Selenium | 50 to 200 µg, |
| Magnesium | 100 to 500 mg, |
| L-Cystine | 50 to 200 mg and |
| Coenzyme Q10 | 5 to 40 mg. |

2. Medicament according to Claim 1, where the amounts per administration unit are:
| | |
|---|---|
| Vitamin B1 | 3 to 7 mg, |
| Vitamin B2 | 3 to 7 mg, |
| Nicotinamide | 30 to 70 mg, |
| Vitamin B6 | 3 to 7 mg, |
| Vitamin C | 1.5 to 3 g, |
| Vitamin E | 400 to 600 I.U., |
| Zinc | 50 to 200 mg, |
| Selenium | 80 to 150 µg, |
| Magnesium | 100 to 500 mg, |
| L-Cystine | 80 to 150 mg and |
| Coenzyme Q10 | 10 to 25 mg. |

## Revendications

1. Médicament pour la prévention de dommages provoqués par un stress oxydant lors de la plongée, contenant, par unité d'administration
| | |
|---|---|
| de la vitamine B1 | 1 à 10 mg, |
| de la vitamine B2 | 1 à 10 mg, |
| du nicotinamide | 10 à 100 mg, |
| de la vitamine B6 | 1 à 10 mg, |
| de la vitamine C | 0,5 à 5 g, |
| de la vitamine E | 100 à 1000 i.E., |
| du zinc | 50 à 200 mg, |
| du sélénium | 50 à 200 µg, |
| du magnésium | 100 à 500 mg, |
| de la L-cystine | 50 à 200 mg et |
| de la coenzyme Q10 | 5 à 40 mg. |

2. Médicament selon 1a revendication 1, les quantités par unité d'administration étant :
| | |
|---|---|
| vitamine B1 | 3 à 7 mg, |
| vitamine B2 | 3 à 7 mg, |
| nicotinamide | 30 à 70 mg, |
| vitamine B6 | 3 à 7 mg, |
| vitamine C | 1,5 à 3 g, |
| vitamine E | 400 à 600 i.E., |
| zinc | 50 à 200 mg, |
| sélénium | 80 à 150 µg, |
| magnésium | 100 à 500 mg, |
| L-cystine | 80 à 150 mg et |
| coenzyme Q10 | 10 à 25 mg. |
